# EUROPÄISCHE PATENTANMELDUNG

(11) **EP 1 376 128 A1**
(43) Veröffentlichungstag der Anmeldung: **02.01.2004**
(21) Anmeldenummer: 03013220.3
(22) Anmeldetag: 12.06.2003
(51) Int. Cl.: G01N 33/543, C12Q 1/00, G01N 27/02

(54) **Methode und Vorrichtung zum impedimetrischen Nachweis eines oder meherer Analyten in einer Probe**

(30) Priorität: 25.06.2002 DE 10228260
(71) Anmelder: Bayer Aktiengesellschaft, 51368 Leverkusen (DE)
(72) Erfinder: Diessel, Edgar, Dr., 51061 Köln (DE); Hoheisel, Werner, Dr., 51061 Köln (DE); Merker, Udo, Dr., 50679 Köln (DE); Burmeister, Jens, Dr., 51061 Köln (DE); Köhler, Burkhard, Dr., 51373 Leverkusen (DE)

(57) **Zusammenfassung**

Die Erfindung betrifft ein Verfahren zum Nachweis eines oder mehrerer Analyten durch eine Erkennungsreaktion, mit den Schritten
- Bereitstellung einer Vorrichtung mit
   - einer Messelektrode mit biofunktioneller Oberfläche, wobei die biofunktionelle Oberfläche Erkennungselemente für die Analyten aufweist,
   - einer oder mehreren Gegenelektroden,
   - einem flüssigen Elektrolyten zwischen Messelektrode und Gegenelektroden,
- in Kontaktbringen von mit elektrisch aktiven Markierungseinheiten markierten Analyten mit der biofunktionellen Oberfläche, wobei die elektrisch aktiven Markierungseinheiten entweder vor dem Kontakt der Analyten mit der biofunktionellen Oberfläche an die Analyten gebunden worden sind oder nach dem Kontakt der Analyten mit der biofunktionellen Oberfläche an die Analyten gebunden werden,
- Anlegen einer a) zeitlich veränderlichen Spannung oder b) eines zeitlich veränderlichen Stroms zwischen der ersten Gegenelektrode und der Messelektrode, und
- entweder im Fall a) Messung des Stroms oder im Fall b) Messung der Spannung zwischen erster Gegenelektrode und Messelektrode, oder
- im Fall a) Messung des Stroms oder im Fall b) Messung der Spannung Messung des Stroms zwischen zweiter oder weiterer Gegenelektrode und der Messelektrode.

Die Erfindung betrifft weiterhin eine Vorrichtung mit der das erfindungsgemäße Verfahren durchgeführt werden kann.

## Beschreibung

Die Erfindung betrifft ein Verfahren und eine Vorrichtung zum qualitativen und/oder quantitativen impedimetrischen Nachweis von Analyten in einer Probe. Hierbei handelt es sich vorzugsweise um die spezifische Detektion eines biologisch relevanten Moleküls in einem wässrigen Medium. Ein derartiges Sensorprinzip bzw. ein solcher Sensor hat ein großes Anwendungsgebiet z.B. in der Umweltanalytik, dem Nahrungsmittelbereich, der Human- und Veterinärdiagnostik, dem Pflanzenschutz und in der biochemischen bzw. pharmakologischen Forschung.

Für derartige diagnostische Anwendungen sind Bio- und Chemosensoren bekannt, die eine biofunktionelle Oberfläche und einen physikalischen Signalwandler aufweisen.

An biofunktionelle Oberflächen werden biologische, chemische oder biochemische Erkennungselemente wie z.B. DNA, RNA, Aptamere, Rezeptoren gebunden, an die ein Analyt beim Nachweis mittels einer Erkennungsreaktion spezifisch bindet.

Beispiele für Erkennungsreaktionen sind die Bindung von Liganden an Komplexe, die Komplexierung von Ionen, die Bindung von Liganden an (biologische) Rezeptoren, Membranrezeptoren oder Ionenkanäle, von Antigenen oder Haptenen an Antikörper (Immunoassays), von Substraten an Enzyme, von DNA oder RNA an bestimmte Proteine, von Aptameren oder Spiegelmeren an ihre Targets, die Hybridisierung von DNA/RNA/PNA oder anderen Nukleinsäure-Analoga (DNA-Assays) oder die Prozessierung von Substraten durch Enzyme.

Beispiele für nachzuweisende Analyten sind DNA, RNA, PNA, Nukleinsäure-Analoga, Enzymsubstrate, Peptide, Proteine, potentielle Wirkstoffe, Medikamente, Zellen, Viren.

Beispiele für Erkennungselemente, an die die nachzuweisenden Analyten binden, sind DNA, RNA, PNA, Nukleinsäure-Analoga, Aptamere, Spiegelmere, Peptide, Proteine, Komplexbildner für Metalle/Metallionen, Cyclodextrine, Kronenether, Antikörper oder deren Fragmente, Anticaline, Enzyme, Rezeptoren, Membranrezeptoren, Ionenkanäle, Zelladhäsionsproteine, Ganglioside, Mono- oder Oligosaccharide.

Erkennungselemente können kovalent oder nichtkovalent an die biofunktionelle Oberfläche gekoppelt werden. Die kovalente Immobilisierung von Erkennungselementen wie z.B. DNA an Sensoroberflächen hat hinsichtlich der Stabilität, Reproduzierbarkeit und Spezifität der Kopplung entscheidende Vorteile gegenüber der nichtkovalenten Kopplung. Einen Überblick über Methoden zur Präparation DNA-beschichteter Oberflächen gibt S. L. Beaucage, Curr. Med. 2001, 8, 1213-1244.

Ein Beispiel für nichtkovalente Kopplung ist das Spotting von cDNA auf Glasträger, an die zuvor Polylysin adsorbiert wurde.

Werden an die Oberfläche des Signalwandlers verschiedene Erkennungselemente räumlich voneinander getrennt gebunden, so kann eine große Zahl von Erkennungsreaktionen mit einer zu untersuchenden Probe zeitgleich durchgeführt werden. Verwirklicht ist dies z.B. bei sogenannten DNA-Arrays, bei denen verschiedene DNA-Sequenzen (z.B. Oligonukleotide oder cDNAs) auf einem festen Träger (z.B. Glas) immobilisiert sind. Solche DNA-Arrays werden in der Regel mit optischen aber auch mit elektrischen Methoden ausgelesen und finden Anwendung in der Expressions-Profilierung, der Sequenzierung, dem Nachweis viraler oder bakterieller Nukleinsäuren oder der Genotypisierung.

Die Detektion der Erkennungsreaktion bei Bio- oder Chemosensoren kann durch Anwendung optischer, elektrischer bzw. elektrochemischer, mechanischer oder magnetischer Signalwandlungsverfahren erfolgen.

Insbesondere die am weitesten fortgeschrittenen beschriebenen optischen Verfahren verfügen zwar über hohe Empfindlichkeiten lassen sich aber aufgrund des komplexen Aufbaus aus Lichtquelle, Sensor und Lichtdetektor in der Regel nur beschränkt miniaturisieren und werden daher den elektrischen Methoden in Bezug auf Herstellungskosten unterlegen bleiben.

Aus diesen Gründen kommt der Entwicklung von elektrischen Sensoren eine wachsende Bedeutung zu. Insbesondere führt der Einsatz von Mikrostrukturierungstechnik aus der Halbleitertechnologie zu miniaturisierten Formaten, die hohe Empfindlichkeiten versprechen. In DE 43 185 19 wie auch in WO 97/21094 werden mikrostrukturierte Elektrodenanordnungen verwendet, um eine spezifische Bindung von unmarkierten Antikörpern oder DNA an Antigene bzw. komplementäre DNA, die zwischen zwei Elektroden immobilisiert sind, mittels Impedanzmessungen nachzuweisen. Insbesondere werden in DE-A 4 318 519 die nachzuweisenden Moleküle mit reversibel reduzierbaren bzw. oxidierbaren Molekülen markiert, so dass in diesen Interdigitalstrukturen durch elektrochemisches Rezyklisieren Verstärkungseffekte erzielt werden.

Eine alternative Methode zur Verstärkung von elektrochemischen Signalen besteht in einer enzyminduzierten Präzipitation von Polymeren, die den Elektronentransferwiderstand signifikant erhöhen (Patolsky et al., Langmuir 15, 3703 (1999)).

Elektrochemische Verfahren können durch unspezifischen Nachweis von elektroaktiven Substanzen, wie sie in realen Proben wie z.B. Körperflüssigkeiten vorhanden sind, beeinträchtigt werden.

Zur Detektion von geladenen Molekülen oder Komplexen aus geladenen Molekülen und Liganden werden Feldeffekttransistoren eingesetzt (US 6,203,981).

Nanopartikel können als alternatives Substratmaterial verwendet werden. In EP-A 1 022 560 A1 wird die Leitfähigkeit eines Nanopartikelnetzwerks durch Ligandadsorption modifiziert. WO 01/13432 A1 offenbart die Verwendung eines einzelnen Nanoteilchens als Einzelelektronentransistor, dessen Strom-Spannungskennlinie durch Ligandadsorption beeinflusst wird.

Bei diesen Konzepten, die auf elektrostatischen Feldeffekten beruhen, kann es in realen Proben wie z.B. Blut, Urin zu Interferenzen zwischen der gewünschten Ligandadsorption und unspezifischen Adsorptionen geladener Moleküle an die Sensoroberfläche kommen.

Diesbezüglich sind Methoden überlegen, die Markierungseinheiten für den Analyten benutzen, deren Eigenschaften sich signifikant von denen der Bestandteile der zu analysierenden Probe unterscheiden. Hierzu bieten sich z.B. metallische Nanopartikel als Markierungseinheiten an.

In US 5,858,666 ist die Anwendung metallischer Nanopartikel als Markierungseinheiten in der elektrischen Biosensorik offenbart. Im Rahmen von Gleichstrommessungen besitzen bestimmte elektrische Biosensoren mit metallischen Nanopartikeln das Potenzial für eine außerordentlich hohe Empfindlichkeit bis in den Einzelmolekülbereich. Dieses Potenzial erschließt sich insbesondere durch autometallografische Abscheidung. Bei diesem sog. Autometallografie-Prozess, der aus der Fotografie und der Elektronenmikroskopie bekannt ist, wirken die Nanopartikel oder Kolloide als Katalysatoren für den Elektronenübertrag von einem Reduktionsmittel auf ein Au- oder Ag-Ion, das sich in Form eines Ag oder Au-Salzes mit dem Reduktionsmittel wie z.B. Hydrochinon in der Verstärkungslösung befindet. Nach erfolgter Reduktion fällt das Ion auf dem Kolloid als Metall aus. Als elektrischer Signalwandler werden hierfür Elektrodenpaare gewählt, die durch eine Isolator voneinander getrennt sind. Mit Nanopartikeln markierte Analytmoleküle bilden mit autometallographischer Verstärkung eine leitfähige Brücke zwischen den Elektroden, die durch eine Gleichstrom-Widerstandsmessung detektiert wird. Die grundlegenden Patente hierzu sind US-A 4,794,089; US-A 5,137,827; US-A 5,284,748. Weitere Offenbarungen finden sich in DE-A 198 60 547, WO 99/57550 und in WO 01/00876. Die Detektion von Nukleinsäuren durch Gleichstrom-Widerstandsmessung wurde demonstriert (Möller et al., Langmuir 17, 5426 (2001)). Als weitere Ausbaustufe dieser Methode wird die Diskriminierung von Punktmutationen (Single Nucleotide Polymorphisms (SNPs)) in (Park et al., Science 295, 1503 (2002)) beschrieben.

In den beiden letztgenannten Ausführungsformen sind die Elektrodenabstände sehr viel größer als die Partikel nach dem Autometallografie-Prozess (ca. Faktor 100-1000). Damit muss sich ein Perkolationsweg zwischen den Elektroden bilden, um einen Stromfluss zu ermöglichen. Dies schränkt den dynamischen Bereich der Messmethode entscheidend ein, so dass diese Methoden in der Regel nur als Schwellwertmethoden eingesetzt werden. Dynamische Bereiche erschließen sich nur durch einen sehr umständlichen mehrfachen autometallografischen Verstärkungsprozess, der sich nicht für den praktischen Einsatz in einem Biosensor empfiehlt.

Der Erfindung liegt die Aufgabe zugrunde, einen hochempfindlichen elektrischen Sensor und eine Messmethode zum Nachweis von Analyten mittels Erkennungsreaktion zu entwickeln, die eine hohe Empfindlichkeit haben und zusätzlich in Bezug auf die Menge der nachzuweisenden Analyten quantifizierbar sind.

Die Lösung der erfindungsgemäßen Aufgabe besteht in einem Verfahren zum Nachweis eines oder mehrerer Analyten durch eine Erkennungsreaktion, mit den Schritten
- Bereitstellung einer Vorrichtung mit
   - einer Messelektrode mit biofunktioneller Oberfläche, wobei die biofünktionelle Oberfläche Erkennungselemente für die Analyten aufweist,
   - einer oder mehreren Gegenelektroden,
   - einem flüssigen Elektrolyten zwischen Messelektrode und Gegenelektroden,
- in Kontaktbringen von mit elektrisch aktiven Markierungseinheiten markierten Analyten mit der biofunktionellen Oberfläche, wobei die elektrisch aktiven Markierungseinheiten entweder vor dem Kontakt der Analyten mit der biofünktionellen Oberfläche an die Analyten gebunden worden sind oder nach dem Kontakt der Analyten mit der biofünktionellen Oberfläche an die Analyten gebunden werden,
- Anlegen einer a) zeitlich veränderlichen Spannung oder b) eines zeitlich veränderlichen Stroms zwischen der ersten Gegenelektrode und der Messelektrode, und
- entweder im Fall a) Messung des Stroms oder im Fall b) Messung der Spannung zwischen erster Gegenelektrode und Messelektrode, oder
- im Fall a) Messung des Stroms oder im Fall b) Messung der Spannung Messung des Stroms zwischen zweiter oder weiterer Gegenelektrode und der Messelektrode.

Erfindungsgemäß sind Erkennungselemente für die Analyten an eine Messelektrode mit biofunktioneller Oberfläche gebunden. Die Analyten gehen mit den Erkennungselementen eine Erkennungsreaktion ein.

Eine zeitlich veränderliche Spannung oder ein zeitlich veränderlicher Strom wird zwischen der Messelektrode und einer Gegenelektrode angelegt. Die zeitlich veränderliche Spannung kann z.B. eine Wechselspannung oder eine gepulste Spannung, der zeitlich veränderliche Strom kann z.B. ein Wechselstrom oder ein gepulster Strom sein.

Beim Anlegen der zeitlich veränderlichen Spannung bzw. des zeitlich veränderlichen Stroms bildet sich an den Elektroden eine Helmholtz-Doppelschicht mit einer bestimmten Impedanz. Die Impedanz dieser Helmholtz-Doppelschicht wird modifiziert, wenn Analyten, die mit einer elektrisch aktiven Markierungseinheit markiert sind, durch die Erkennungsreaktion an die biofunktionelle Oberfläche gebunden werden, da z.B. durch die elektrisch aktiven Markierungseinheiten die Fläche der Messelektrode insbesondere durch einen elektrischen Kontakt zwischen leitfähigen Markierungseinheiten und der Messelektrode vergrößert wird.

Der Analyt kann vor der Bindung an das Erkennungselement bereits mit einer elektrisch aktiven Markierungseinheit markiert sein oder er wird erst nach der Bindung an das Erkennungselement markiert, indem z.B. ein Bindungselement, das mit einer Markierungseinheit markiert ist, an den Komplex bestehend aus Erkennungselement und Molekül bindet.

Mit dem erfindungsgemäßen Verfahren lässt sich die Modifikation der Impedanz durch eine einzige Markierungseinheit, das heißt in der Regel durch einen einzigen markierten Analyten nachweisen. Jede Markierungseinheit trägt unabhängig von anderen Markierungseinheiten zu einem Messsignal bei. Außerdem können Analytmoleküle mit mehreren Markierungseinheiten versehen werden, um die Empfindlichkeit der Methode noch zusätzlich zu steigern.

Die Immobilisierung der Erkennungseinheiten an der Oberfläche der Messelektrode erfolgt durch dem Fachmann bekannten Methoden nach dem Stand der Technik. Für DNA-Erkennungseinheiten ist diese Immobilisierung z.B. in S. L. Beaucage, Curr. Med. 2001, 8, 1213-1244 beschrieben.

Für die Immobilisierung auf der Elektrodenoberfläche wird eine optimale Dichte von Erkennungseinheiten angestrebt, die bei hoher Flächendichte eine optimale Aktivität der Erkennungseinheit gewährleistet.

Die Immobilisierung von Erkennungselementen wie Antikörpern kann kovalent oder nichtkovalent erfolgen. Beispielsweise können Avidin oder Streptavidin auf der Oberfläche physisorbiert oder nach geeigneter Biofunktionalisierung der Oberfläche kovalent immobilisiert werden. An die mit Avidin oder Streptavidin beschichtete Oberfläche können beispielsweise biotinylierte Antikörper spezifisch immobilisiert werden.

Aus den Impedanzmessungen kann die Kapazität der Doppelschicht unter der Verwendung von geeigneten Ersatzschaltbildern rechnerisch abgeleitet werden.

Um den Arbeitspunkt der Impedanzmessung einzustellen, kann eine Gleichspannung bzw. ein Gleichstrom auf die zeitlich veränderliche Spannung bzw. den zeitlich veränderlichen Strom aufgeprägt werden.

Das erfindungsgemäße Verfahren kann z.B. für einen Immuno- oder DNA-Assay eingesetzt werden. DNA-Assays dienen bevorzugt zur Detektion von viraler DNA oder RNA oder von DNA bakterieller Spezies, sowie zur Expressions-Profilierung, zur Genotypisierung für die Diagnose von Erbkrankheiten oder für Pharmacogenomics (genetisch bedingte Wirkweise bzw. Nebenwirkungen von Pharmaka), Nutrigenomics (genetisch bedingte Wirkweise bzw. Nebenwirkungen von Nahrungsmitteln). Bei der Genotypisierung werden insbesondere Veränderungen von Genen festgestellt, die nur auf der Variation einer Base beruhen (single nucleotide polymorphism = SNP).

Die Analyten können durch die Erkennungsreaktion auch indirekt nachgewiesen werden. Bei der indirekten Detektion werden Analyten, die bereits vor Bindung an das Erkennungselement mit Markierungseinheiten markiert sind, in Kontakt mit der biofunktionellen Oberfläche gebracht. Gleichzeitig werden zusätzlich unmarkierte Analyten in Kontakt mit der biofunktionellen Oberfläche gebracht. Diese beiden Spezies kompetieren um die Bindung an die immobilisierten Erkennungselemente. Befinden sich in dem Elektrolyten zwischen Messelektrode und Gegenelektrode keine unmarkierten Analyten, so werden alle Bindungsplätze an den Erkennungselementen von markierten Analyten besetzt und die Modifikation der Impedanz wird maximal. Für eine nichtverschwindende Konzentration von unmarkierten Analyten werden die Bindungsplätze an den Erkennungselementen entsprechend der jeweiligen Konzentrationen von unmarkierten Analyten und markierten Analyten anteilig besetzt, so dass die Modifikation der Impedanz im Vergleich zur verschwindenden Konzentration des unmarkierten Analytens kleiner ist.

Nach dem erfindungsgemäßen Verfahren werden Analyten mit Markierungseinheiten markiert, die in geeigneter Weise elektrisch aktiv sind.

Die elektrische Aktivität kann in der elektrischen Leitfähigkeit des für die Markierungseinheiten verwendeten Materials bestehen, die vorzugsweise im Bereich metallischer Leitfähigkeiten liegt.

Als elektrisch aktive Markierungseinheiten können Nanopartikel, Metallkomplexe und/oder Cluster aus leitfähigen Materialien wie Au, Ag, Pt, Pd, Cu oder Kohlenstoff eingesetzt werden.

Die elektrische Aktivität kann aber auch in den dielektrischen Eigenschaft des für die Markierungseinheiten verwendeten Materials bestehen. Die Dielektrizitätskonstante der Markierungseinheit liegt vorzugsweise im Bereich von 5 bis 15000, besonders bevorzugt im Bereich zwischen 10 und 1500.

Die Größe der elektrisch aktiven Markierungseinheiten liegt bevorzugt im Bereich zwischen 1 und 100 nm, bevorzugt im Bereich zwischen 1 und 30 nm und besonders bevorzugt im Bereich von 1 bis 2 nm. Ganz besonders bevorzugt sind Au-Cluster bestehend aus 50 bis 150 Atomen mit einer Größe im Bereich von 1 bis 2 nm. Die Größeangabe bezieht sich dabei auf den größten Durchmesser der Markierungseinheiten.

Markierungseinheiten mit hohen Dielektrizitätskonstanten können Nanopartikel oder Cluster aus Titanaten, Materialien, die in einem Perovskitgitter kristallisieren, TiO₂ oder Bleiverbindungen sein. Diese haben häufig eine Größe im Bereich von 1 bis 100 nm. Beispielhaft erreicht PbSO4 eine Dielektrizitätskonstante von 14 bei 100 MHz bzw. BaTiO3 3600 bei 100 kHz. Bei einem Vergleich sind die jeweiligen Frequenzabhängigkeiten zu berücksichtigen.

Weiterhin können Kohienstoff "nanotubes", nichtleitfähige Partikel mit leitfähiger Beschichtung oder nichtleitfähige Partikel mit metallischer Beschichtung als Markierungseinheiten eingesetzt werden. Nichtleitfähige Partikel können z.B. Polystyrolbeads sein. Bei Kohlenstoff "nanotubes" können die Leitfähigkeitseigenschaften gezielt eingestellt werden.

Markierungseinheiten können auch aus leitfähigen Polymeren wie Polyaniline, Polythiophene insbesondere Polyethylendioxythiophen, Polyphenylene, Polyphenylenvinylen, Polythiophenvinylen, Polypyrrole bestehen.

Als Markierungseinheit können auch Enzyme wie z.B. Meerrettichperoxidase (HRP) dienen. HRP induziert die Polymerisierung von Monomeren (Substrat) elektrisch leitfähiger Polymere wie z.B. Polyanilin.

Eine weitere erfindungsgemäße Anwendung von HRP ist die Abscheidung eines Polymers, an das z.B. Nanopartikel oder alle oben beschriebenen Markierungseinheiten direkt oder indirekt über Biotin-Streptavidin, Biotin-Avidin oder Biotin-NeutrAvidin® (NeutrAvidin® , Hersteller: Pierce Biotechnology, Rockford, IL, U.S.A.) gebunden werden. Für den indirekten Fall ist das Polymer biotinyliert. Dieses Prinzip ist als Catalyzed Reporter Deposition (CARD) bekannt.

Besonders vorteilhaft zur Erzielung hoher Empfindlichkeiten ist eine geeignete Verstärkung der Markierungseinheiten wie sie z.B. durch die autometallografische Verstärkung von Metallkolloiden wie Ag oder Au erzielt werden kann.

Die Detektion des Analyten wird in wässrigem Medium als Elektrolyt durchgerührt. Bevorzugt als wässrige Medien sind Körperflüssigkeiten wie Blut, Urin, interstitielle Flüssigkeit und Tränenflüssigkeit.

Gegenstand der Erfindung ist weiterhin eine Vorrichtung zum Nachweis eines oder mehrerer Analyten durch eine Erkennungsreaktion enthaltend
- mindestens eine Messelektrode mit biofunktioneller Oberfläche, wobei die biofunktionelle Oberfläche Erkennungselemente für die Analyten aufweist,
- eine oder mehrere Gegenelektroden,
- einen flüssigen Elektrolyten zwischen Messelektrode und Gegenelektroden,
- Analyten, die mit elektrisch aktiven Markierungseinheiten markiert sind und in Kontakt mit den Erkennungselementen der biofunktionellen Oberfläche gebracht werden können,
- entweder a) eine Spannungsquelle zum Aufprägen einer zeitlich veränderlichen Spannung oder b) eine Stromquelle zum Aufprägen eines zeitlich veränderlichen Stroms zwischen der ersten Gegenelektrode und der Messelektrode, und
- Messgerät zur
   - Messung im Fall a) des Stroms oder im Fall b) der Spannung zwischen erster Gegenelektrode und Messelektrode, oder
   - Messung im Fall a) des Stroms oder im Fall b) der Spannung zwischen zweiter oder weiterer Gegenelektrode und der Messelektrode.

In der erfindungsgemäßen Vorrichtung weisen die Messelektrode, Gegenelektroden, Elektrolyten, Erkennungslemente, Analyten und die elektrisch aktiven Markierungseinheiten bevorzugt die zum Verfahren beschriebenen Eigenschaften auf.

Die Oberfläche der Messelektrode kann in mehrere leitfähige Bereiche eingeteilt sein.

Erfindungsgemäße Elektroden können planar oder in nichtplanaren Geometrien ausgeführt werden.

Hohe Empfindlichkeiten bis in den Einzelmolekülbereich versprechen impedimetrische Messungen auf der Basis von Mikroelektroden mit Flächen im Bereich von 1 bis 20 x 1 bis 20 µm², bevorzugt von 5 bis 15 x 5 bis 15 µm² und besonders bevorzugt von 10 x 10 µm², bei denen einzelne Markierungseinheiten wie z.B. autometallografisch verstärkte Au-Kölloide Vergrößerungen der Elektrodenflächen in der Höhe einiger Prozent bewirken. Mit Einzelelektrodenflächen von z.B. 10 x 10 µm² lassen sich auf einem 10 x 10 mm² großen Chip 10⁶ Elemente unterbringen. Diese Größenangaben haben lediglich Beispielcharakter und schließen andere Größen und Anzahlen nicht aus.

In jedem leitfähigen Bereich kann eine Art von Erkennungselementen immobilisiert sein, oder es können in mehreren leitfähigen Bereichen dieselbe Art von Erkennungselementen immobilisiert sein.

Zur Abdeckung von dynamischen Bereichen, die über einen zu erwartenden Quantifizierungsbereich von zwei bis drei Größenordnungen einer impedimetrischen Messung einer einzelnen Elektrode hinausgehen, werden dementsprechend verschieden große Elektrodenflächen eingesetzt, die sich in ihrer Fläche in dem Verhältnis der nachzuweisenden Konzentrationsbereiche unterscheiden. Dabei werden jeweils für eine Art von Erkennungseinheit mehrere leitfähige Bereiche eingesetzt, die sich in ihrer Größe jeweils um einen Faktor unterscheiden, bevorzugt um einen Faktor im Bereich von 5 bis 15, besonders bevorzugt von 9 bis 11.

In der planaren Ausführung befinden sich eine oder mehrere Elektroden seitlich nebeneinander auf einem Substrat. Analytlösungen können über Mikrokanäle, die in die Strukturen geätzt werden können, den elektrischen Sensorfeldern zugeführt werden. Alternativ kann ein mit Mikrokanälen versehenes Bauteil als Deckel für ein planares Substrat eingesetzt werden.

Ein Beispiel für nichtplanare Geometrien ist ein Substrat, in das z.B. mit Trockenätzverfahren Kanäle vertikal geätzt werden. Die Wände dieser Mikrokanäle werden mit Elektroden überzogen. In diese mikrofluidischen Kanäle können die Analytlösungen in die unmittelbare Nähe der Elektroden gebracht werden, womit die Ansprechzeit bzw. Empfindlichkeit der Vorrichtung aufgrund reduzierter Diffusionswege und -zeiten der Analytmoleküle verringert wird.

Besonders vorteilhaft können mehrere Elektroden lateral nebeneinander oder vertikal übereinander in Form von Schichtstrukturen ausgerührt werden.

Vorteilhaft kann die Gegenelektrode auf dem gleichen Substrat wie die Messelektroden untergebracht sein z.B. für 2-Punkt-Impedanzmessungen. Ebenso können neben Mess-und Gegenelektrode eine zusätzliche Referenzelektrode auf dem gleichen Substrat für eine 3-Punkt-Impedanzmessung aufgebracht werden.

Die Substrate bestehen aus Glas, SiO₂, Kunststoffe, bevorzugt aus Polyethylenterephtalat, Polycarbonat, Polystyrol.

Für die Elektroden kommen Metalle wie z.B. Au, Pl, Ag, Ti, Halbleiter wie z.B. Si, Metalloxide insbesondere Indium-Zinn-Oxid (ITO) oder leitfähige Polymere wie Polyaniline, Polythiophene insbesondere Polyethylendioxythiophen, Polyphenylene, Polyphenylenvinylen, Polythiophenvinylen, Polypyrrole in Frage.

Um viele Elektroden einzeln anzusteuern, werden Multiplexschaltkreise eingesetzt.

Mit einer Impedanzmessung, die mit Wechselspannungen bzw. -strömen arbeitet, setzt sich die erfindungsgemäße Lösung vom unmittelbaren Stand der Technik (Gleichstromdetektion) durch die intrinsisch gegebene Möglichkeit zur Quantifizierung der nachzuweisenden Analyten ab.

Aufgrund der möglichen hohen Packungsdichte der Funktionselemente auf der Messelektrode, die auch als Chip bezeichnet werden kann, eignet sich die erfindungsgemäße Vorrichtung als Plattform für DNA- und Proteinarrays.

### Figuren und Beispiele

Die Figuren zeigen
- Fig. 1: Erkennungsreaktion auf einer Messelektrode
- Fig. 2: Vorrichtung zur Detektion von DNA auf ITO-Elektroden
- Fig. 3: Impedanzspektren einer Hybridisierungsreaktion
- Fig. 4: Vertikale Anordnung der Elektrodenstruktur
- Fig. 5: Vertikale Anordnung von Elektroden-Isolatorschichtfolgen
- Fig. 6: Planare Elektroden in Arrayform

### Beispiel 1

### Methode und Vorrichtung zur Detektion von DNA auf ITO-Elektroden

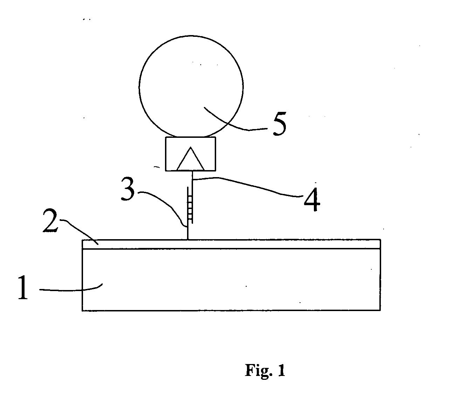

Das elektronische Bauelement als Plattform für die Erkennungsreaktion basiert auf ITO (Indium-Zinn-Oxid)-beschichteten Glasträgern 1 (Merck, 9R1507, Ohm/square: 13, ITO-Schichtdicke: 125nm) (Fig 1), nachfolgend Chips genannt.

An die ITO-Oberflächen 2 wurden wie folgt Fänger-DNA 3 gebunden. 200g L-Lysin, 50g Caprolactam, 50g 1,6-Diaminohexan und 0,5g TPP wurden bei 240°C zur Reaktion gebracht, wobei Wasser abdestillierte. Das resultierende Polyamid wurde im Verhältnis 8:1 mit NMP verdünnt. 9g des Polymers wurden zur Silanisierung 2 h unter N₂-Atmosphäre mit 0,1 g Triethoxysilylpropylisocyanat bei RT umgesetzt, wobei das Silan über Urethangruppen mit den Aminogruppen des Polyamids reagiert. Indium-Zinn-Oxid beschichtete Glasoberflächen wurden 30 min mit Argon-induziertem Plasma bei Normaldruck behandelt und anschließend 5 min auf 80°C erhitzt. Eine 1%ige Lösung des silanfunktionellen Polyamidurethans in einem Gemisch von Aceton/DMF/Wasser (7,5:2:0,5 v/v/v) wurde 15 min bei RT mit den Chips inkubiert. Nach der Funktionalisierung wurden die Oberflächen mit Aceton gewaschen und anschließend 45 min. bei 110°C getrocknet.

Fänger-DNA 3 (5'-Amino--GTCCCCTACGGACAAGGCGCGT-3') wurde in Phosphatpuffer pH 7,2 gelöst und mit 0,1M Bis-sulfo-succinimidyl-suberat (BS3) 10 min bei RT inkubiert. Durch Verdünnung mit Phosphatpuffer wurde die Reaktion abgebrochen. Die Reinigung der Fänger-DNA erfolgte durch Chromatographie auf einer NAP-10-Säule (Pharmacia). Die gereinigte Fänger-DNA wurde in Volumina von z.B. 25 µl auf die silanisierten Oberflächen aufgebracht und über Nacht bei RT inkubiert. Die resultierenden DNA-Chips wurden mit 1% Ammoniumhydroxid sowie Wasser gewaschen und anschließend bei RT getrocknet. Durch eine Übernacht-Inkubation mit 0,4mg/ml BS3 in 0,1M Phosphatpuffer pH 7,2 wurden die nicht umgesetzten Aminogruppen auf der Chipoberfläche blockiert.

Auf den mit Fänger-DNA beschichteten Chipoberflächen wurden DNA-Hybridisierungsreaktionen mit einer Analyt-DNA-Probe 4 durchgeführt. Als Positivkontrolle diente der match-DNA-Analyt der Sequenz 5'-Biotin-TTTTTCGCGCCTTGTCCGTAGGGGACT-3'. Als Negativkontrolle fungierte der complete-mismatch-Analyt der Sequenz 5'-Biotin-GTCCCCTACGGACAAGGCGCGT-3'. Es wurden 10-9 M Lösungen der DNA's in Tris-Puffer pH 8, 1M NaCl, 0,005% SDS, mit dem jeweiligen Chip in einem Volumen von 25 µl für 0.5h bei 56°C inkubiert. Anschließend wurde mit Hybridisierungspuffer gewaschen, um nicht hybridisierte DNA von der Chipoberfläche zu entfernen. Die hybridisierten Target-DNAs wurden 4h bei RT mit einer Lösung von Streptavidin-Gold 5 (Durchmesser der Goldpartikel 10 nm, Sigma) inkubiert. Die Chips wurden mit Wasser gewaschen und anschließend bei RT getrocknet. Die Goldmarkierten Nukleinsäuren wurden mit der Enhancer-Lösung der Fa. Biocell (Biocell L15) einmal für 5 min. bei Raumtemperatur behandelt und anschließend getrocknet.

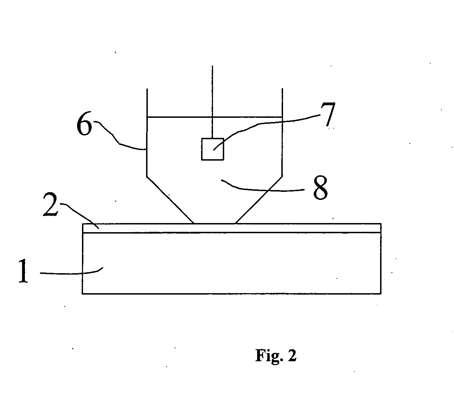

Die impedimetrischen Messungen |Z(ω)| (Betrag des komplexen Widerstands) der Hybridisierungsreaktionen wurde in 2-Punkt-Geometrie über einen Frequenzbereich zwischen 0.1 Hz und 100 kHz bei einer vorgegebenen Wechselspannungsamplitude von 5 mV mit einem EG&G Potentiostat/Galvanostat Model 283 gemessen. Hierzu wurde auf den Chip 1 ein bodenfreier Teflontopf 6 mit einer Bohrung von 1.6 mm² aufgesetzt, der eine Elektrodenfläche von 2 mm² definiert (Fig 2). Während die beschichtete ITO-Elektrode 2 die Messelektrode darstellt, dient eine poröse Tantalelektrode 7 mit einer gesamten Oberfläche von ca. 250 cm² als Gegenelektrode. Als Elektrolyt 8 wurde 0.5 M NaCl verwendet.

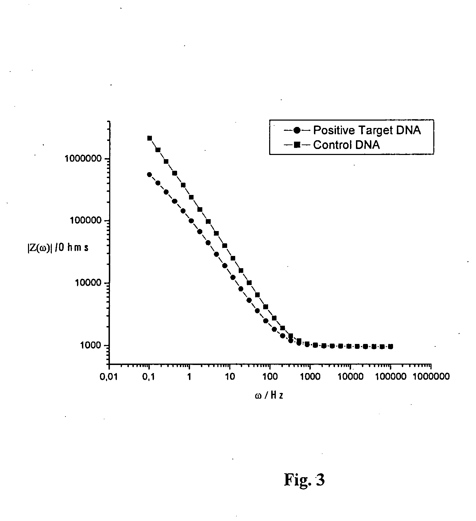

Fig. 3 zeigt die Impedanzspektren für die Hybridisierungsreaktion der Fänger-DNA mit der positiven Analyt-DNA und der Kontrollhybridisierungsreaktion. Für die positiven Reaktion lässt sich eine signifikante Erniedrigung von |Z(ω)| messen.

### Beispiel 2

### Methode und Vorrichtung zur Detektion von DNA mit vertikalen angeordneten Elektrodenstrukturen in Mikrokanälen

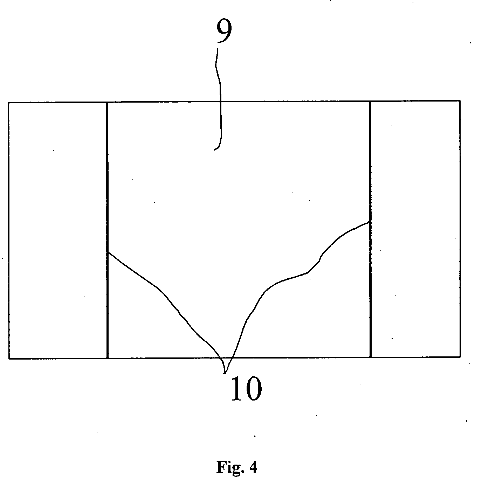

Eine alternative Ausführung für ein erfindungsgemäßes elektronisches Bauelement ist eine vertikale Anordnung einer Elektrodenstruktur nach Fig. 4. Mittels Fotolithografie wird ein Mikrokanal 9 einer Weite von z.B. 20 µm durch Ionenstrahlätzen durch die Schichtstruktur realisiert. Ein anschließender elektrochemischer metallischer Abscheidungsprozess führt zur Metallisierung 10 des Kanals, der mit seiner gesamten Innenfläche als Messelektrode somit zur Verfügung steht. Immobilisierung und Durchführung des Assays finden an der Innenseite dieses Mikrokanals analog zu Beispiel 1 statt.

### Beispiel 3

### Methode und Vorrichtung zur Detektion von DNA mit vertikalen übereinander angeordneten Elektrodenstrukturen

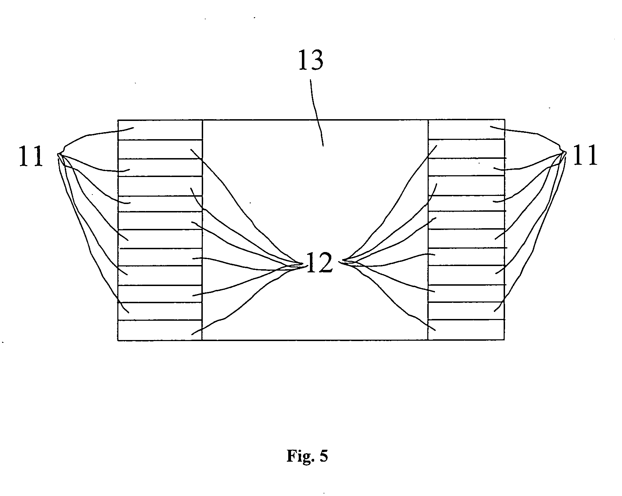

Eine alternative Ausführung für ein erfindungsgemäßes elektronisches Bauelement ist eine vertikale Anordnung von Elektroden-Isolatorschichtfolgen nach Fig. 5. Über mehrstufige Aufdampf- bzw. Sputtersprozesse werden alternierende Schichten von Elektroden 11 und Isolatorschichten 12 übereinander abgeschieden. Mittels Fotolithografie wird ein Mikrokanal 13 einer Weite von z.B. 20 µm durch lonenstrahlätzen durch die Schichtstruktur realisiert. Immobilisierung und Durchführung des Assays finden an der Innenseite dieses Mikrokanals analog zu Beispiel 1 statt. Werden auf den unterschiedlichen Elektroden selektiv verschiedene Fänger-DNA immobilisiert, wird mit diesem Aufbau ein multiplexfähiger Mikrokanal für die impedimetrische Analyse realisiert.

### Beispiel 4

### Methode und Vorrichtung zur Detektion von DNA mit planaren Elektroden in Arrayform und Multiplexeinrichtung

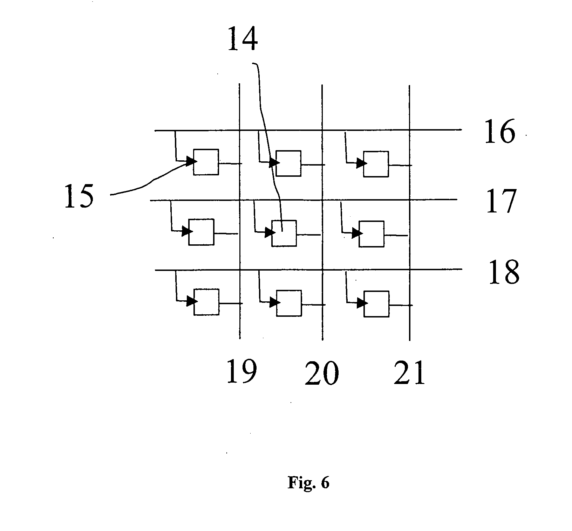

Die Sensoroberfläche besteht aus einem Netzwerk von einzelnen elektronischen Bauelementen 14 nach Beispiel 1 bzw. Beispiel 2, die untereinander über nichtlineare Elemente wie z.B. Dioden 15 und Steuerleitungen 16 - 21 verknüpft sind (Fig. 6), Immobilisierung und Durchführung des Assays finden an der Innenseite dieses Mikrokanals analog zu Beispiel 1 statt. Zum Auslesen eines einzelnen Bauelements 14 werden die Zeilen-Steuerleitungen 17 gegenüber den Spalten-Steuerleitungen 20 auf eine Durchlassspannung gesetzt. Gleichzeitig werden die zu den übrigen Bauelementen zugehörigen Zeilen- und Spaltensteuerleitungspaare 16/19, 16/20, 16/21, 17/19, 17/21, 18/19, 18/20, 18/21 auf die inverse Spannung bzw. Sperrspannung gesetzt. N x N Bauelemente werden über 2 x N Steuerleitungen angesteuert. Die elektrischen Ansteuerungen dieser Leitungen werden durch Standardmultiplexschaltungen bewerkstelligt.

SEQUENCE LISTING

<120> Methode für die impedimetrische Detektion eines oder mehrerer Analyten
in einer Probe und eine Vorrichtung zur Durchführung dieser Methode

<130> 100717-

<140> Nicht verfügbar
<141> Nicht verfügbar

<150> DE 10228260
<151> 2002-06-25

<160> 2

<170> Microsoft Word

<210> 1
<211> 22
<212> DNA
<213> Sequenz des künstlichen Analytens
<220>
<221> misc_feature ? (weitere Eigenschaft)
<222> (1) . . (22)
<223> Beschreibung der Sequenz des künstlichen Analytens

<400> 1
GTCCCCTACGGACAAGGCGCGT

<210> 2
<211> 27
<212> DNA
<213> Sequenz des künstlichen Analytens

<220>
<221> misc_feature ? (weitere Eigenschaft)
<222> (1) . . (27)
<223> Beschreibung der Sequenz des künstlichen Analytens

<400> 2
TTTTTCGCGCCTTGTCCGTAGGGGACT

## Patentansprüche

1. Verfahren zum Nachweis eines oder mehrerer Analyten durch eine Erkennungsreaktion, mit den Schritten
- Bereitstellung einer Vorrichtung mit
- einer Messelektrode mit biofünktioneller Oberfläche, wobei die biofunktionelle Oberfläche Erkennungselemente für die Analyten aufweist,
- einer oder mehreren Gegenelektroden,
- einem flüssigen Elektrolyten zwischen Messelektrode und Gegenelektroden
- in Kontaktbringen von mit elektrisch aktiven Markierungseinheiten markierten Analyten mit der biofunktionellen Oberfläche, wobei die elektrisch aktiven Markierungseinheiten entweder vor dem Kontakt der Analyten mit der biofunktionellen Oberfläche an die Analyten gebunden worden sind oder nach dem Kontakt der Analyten mit der biofunktionellen Oberfläche an die Analyten gebunden werden,
- Anlegen einer a) zeitlich veränderlichen Spannung oder b) eines zeitlich veränderlichen Stroms zwischen der ersten Gegenelektrode und der Messelektrode, und
- entweder im Fall a) Messung des Stroms oder im Fall b) Messung der Spannung zwischen erster Gegenelektrode und Messelektrode, oder
- im Fall a) Messung des Stroms oder im Fall b) Messung der Spannung Messung des Stroms zwischen zweiter oder weiterer Gegenelektrode und der Messelektrode.

2. Verfahren nach Anspruch 1, **dadurch gekennzeichnet, dass** die Erkennungselemente kovalent oder nichtkovalent auf dem elektronischen Bauelement immobilisiert sind

3. Verfahren nach Anspruch 1 oder 2, **dadurch gekennzeichnet, dass** die zeitlich veränderliche Spannung eine Wechselspannung oder eine gepulste Spannung ist.

4. Verfahren nach Anspruch 1 oder 2, **dadurch gekennzeichnet, dass** der zeitlich veränderliche Strom ein Wechselstrom oder ein gepulster Strom ist.

5. Verfahren nach einem der Ansprüche 1 bis 4, **dadurch gekennzeichnet, dass** die Impedanz zwischen der Messelektrode und der ersten oder weiteren Gegenelektrode bestimmt wird.

6. Verfahren nach Anspruch 5, **dadurch gekennzeichnet, dass** die Kapazität zwischen der Messelektrode und der ersten oder weiteren Gegenelektroden aus der Impedanzmessung unter der Verwendung von geeigneten Ersatzschaltbildern abgeleitet wird.

7. Verfahren nach einem der Ansprüche 1 bis 3, **dadurch gekennzeichnet, dass** der zeitlich veränderlichen Spannung eine Gleichspannung aufgeprägt wird.

8. Verfahren nach einem der Ansprüche 1, 2 oder 4, **dadurch gekennzeichnet, dass** dem zeitlich veränderlichen Strom ein Gleichstrom aufgeprägt wird.

9. Verfahren nach einem der Ansprüche 1 bis 8, **dadurch gekennzeichnet, dass** die Erkennungsreaktion einen Immunoassay, DNA-Assay, bevorzugt einen SNP-Assay darstellt.

10. Verfahren nach einem der Ansprüche 1 bis 9, **dadurch gekennzeichnet, dass** die elektrisch aktiven Markierungseinheiten vor dem Kontakt der Analyten mit der biofunktionellen Oberfläche an die Analyten gebunden worden sind und zusätzlich unmarkierte Analyten in Kontakt mit der biofunktionellen Oberfläche gebracht werden.

11. Verfahren nach einem der Ansprüche 1 bis 10, **dadurch gekennzeichnet, dass** ein Analytmolekül mit mehreren elektrisch aktiven Markierungseinheiten markiert ist.

12. Verfahren nach einem der Ansprüche 1 bis 11, **dadurch gekennzeichnet, dass** die elektrisch aktiven Markierungseinheiten eine Dielektrizitätskonstante im Bereich von 5 und 15000, bevorzugt im Bereich zwischen 10 und 1500 aufweisen.

13. Verfahren nach einem der Ansprüche 1 bis 12, **dadurch gekennzeichnet, dass** die elektrisch aktiven Markierungseinheiten eine Größe im Bereich von 1 bis 100 nm, bevorzugt 1 bis 30 nm, besonders bevorzugt von 1 bis 2 nm haben.

14. Verfahren nach einem der Ansprüche 1 bis 13, **dadurch gekennzeichnet, dass** die elektrisch aktiven Markierungseinheiten Nanopartikel, Metallkomplexe und/oder Cluster aus leitfähigen Materialien wie Au, Ag, Pt, Pd, Cu oder Kohlenstoff sind.

15. Verfahren nach Anspruch 14, **dadurch gekennzeichnet, dass** die Nanopartikel oder Cluster aus Titanaten, Materialien, die in einem Perovskitgitter kristallisieren, TiO2 oder Bleiverbindungen sind.

16. Verfahren nach einem der Ansprüche 1 bis 13, **dadurch gekennzeichnet, dass** die elektrisch aktiven Markierungseinheiten Kohlenstoff _{"}nanotubes", nichtleitfähige Partikel mit leitfähiger Beschichtung oder nichtleitfähige Partikel mit metallischer Beschichtung sind.

17. Verfahren nach einem der Ansprüche 1 bis 13, **dadurch gekennzeichnet, dass** die elektrisch aktiven Markierungseinheiten leitfähige Polymere sind.

18. Verfahren nach Anspruch 17, **dadurch gekennzeichnet, dass** die leitfähigen Polymere Polyaniline, Polythiophene insbesondere Polyethylendioxythiophen, Polyphenylene, Polyphenylenvinylen, Polythiophenvinylen, Polypyrrol sind.

19. Verfahren nach einem der Ansprüche 1 bis 13, **dadurch gekennzeichnet, dass** die Markierungseinheiten Enzyme, bevorzugt HRP sind, die durch die Umsetzung eines Substrats elektrisch aktive Markierungseinheiten bilden.

20. Verfahren nach Anspruch 19, **dadurch gekennzeichnet, dass** Meerrettichperoxidase (HRP) die Polymerisierung eines leitfähigen Polymers, bevorzugt Polyanilins oder Polyethylendioxythiophen, katalysiert oder die Abscheidung eines biotinylierten Polymers katalysiert, an deren Biotine Markierungseinheiten über Avidin, NeutrAvidin oder Streptavidin gebunden werden können.

21. Verfahren nach Anspruch 20, **dadurch gekennzeichnet, dass** die elektrisch aktiven Markierungseinheiten autometallografisch verstärkt werden.

22. Verfahren nach Anspruch 21, **dadurch gekennzeichnet, dass** zur autometallografischen Verstärkung Ag oder Au verwendet wird.

23. Vorrichtung zum Nachweis eines oder mehrerer Analyten durch eine Erkennungsreaktion enthaltend
- mindestens eine Messelektrode mit biofunktioneller Oberfläche, wobei die biofunktionelle Oberfläche Erkennungselemente für die Analyten aufweist,
- eine oder mehrere Gegenelektroden,
- einen flüssigen Elektrolyten zwischen Messelektrode und Gegenelektroden,
- Analyten, die mit elektrisch aktiven Markierungseinheiten markiert sind und in Kontakt mit den Erkennungselementen der biofünktionellen Oberfläche sind,
- entweder a) eine Spannungsquelle zum Aufprägen einer zeitlich veränderlichen Spannung oder b) eine Stromquelle zum Aufprägen eines zeitlich veränderlichen Stroms zwischen der ersten Gegenelektrode und der Messelektrode, und
- Messgerät zur
- Messung im Fall a) des Stroms oder im Fall b) der Spannung zwischen erster Gegenelektrode und Messelektrode, oder
- Messung im Fall a) des Stroms oder im Fall b) der Spannung zwischen zweiter oder weiterer Gegenelektrode und der Messelektrode.

24. Vorrichtung nach Anspruch 23, **dadurch gekennzeichnet, dass** die Messelektrode, Gegenelektroden, Elektrolyten, Erkennungslemente, Analyten, zeitlich veränderliche Spannung, der zeitlich veränderlicher Strom und die elektrisch aktiven Markierungseinheiten die in den Ansprüchen 2 bis 4 oder 7 bis 22 beschriebenen Eigenschaften aufweisen.

25. Vorrichtung nach Anspruch 23, 24, **dadurch gekennzeichnet, dass** die Oberfläche der Messelektrode in mehrere leitfähige Bereiche eingeteilt ist.

26. Vorrichtung nach Anspruch 25, **dadurch gekennzeichnet, dass** die leitfähigen Bereiche planar ausgeführt sind.

27. Vorrichtung nach Anspruch 25, 26, **dadurch gekennzeichnet, dass** die leitfähigen Bereiche Größen im Bereich von 1 bis 20 x 1 bis 20 µm², bevorzugt von 5 bis 15 x 5 bis 15 µm², besonders bevorzugt von 10 x 10 µm² haben.

28. Vorrichtung nach einem der Ansprüche 25 bis 27, **dadurch gekennzeichnet, dass** in jedem leitfähigen Bereich eine Art von Erkennungselementen immobilisiert ist.

29. Vorrichtung nach einem der Ansprüche 25 bis 27, **dadurch gekennzeichnet, dass** in mehreren leitfähigen Bereichen dieselbe Art von Erkennungselementen immobilisiert sind.

30. Vorrichtung nach einem der Ansprüche 25 bis 27, **dadurch gekennzeichnet, dass** jeweils für eine Art von Erkennungseinheiten mehrere leitfähige Bereiche eingesetzt werden, die sich in ihrer Größe jeweils um einen Faktor unterscheiden, bevorzugt um einen Faktor im Bereich von 5 bis 15, besonders bevorzugt von 9 bis 11.

31. Vorrichtung nach einem der Ansprüche 25 bis 30, **dadurch gekennzeichnet, dass** das die leitfähigen Bereiche als Kanäle in einem Substrat ausgeführt sind.

32. Vorrichtung nach einem der Ansprüche 25 bis 30, **dadurch gekennzeichnet, dass** mehrere Elektroden lateral nebeneinander oder vertikal übereinander in Form von Schichtstrukturen ausgeführt werden.

33. Vorrichtung nach einem der Ansprüche 25 bis 30, dadurch gekenrizeichnet, dass das die leitfähigen Bereiche in einer alternierenden Schichtfolge von leitfähigen und Isolatorschichten als Mikrokanal in einem Substrat ausgeführt sind.

34. Vorrichtung nach einem der Ansprüche 25 bis 30, **dadurch gekennzeichnet, dass** die Gegenelektroden bzw. Gegenelektrode und Referenzelektrode auf dem gleichen Substrat wie die Messelektroden untergebracht ist.

35. Vorrichtung nach Anspruch 34, **dadurch gekennzeichnet, dass** die Substrate aus Glas, SiO₂, Kunststoffe, bevorzugt Polyethylenterephtalat, Polycarbonat, Polystyrol bestehen.

36. Vorrichtung nach einem der Ansprüche 23 bis 35, **dadurch gekennzeichnet, dass** die leitfähigen Bereichen aus Metallen, bevorzugt Au, Pt, Ag, Ti, Halbleitern wie Si, oder Metalloxiden, bevorzugt Indium-Zinn-Oxid oder leitfähigen Polymeren wie Polyethylendioxythiophen, Polyphenylene, Polyphenylenvinylen, Polythiophenvinylen, Polypyrrol bestehen.

37. Vorrichtung nach einem der Ansprüche 23 bis 36, **dadurch gekennzeichnet, dass** die Messelektroden ein Array bilden.

38. Verwendung der Vorrichtung nach einem der Ansprüche 23 bis 38 als DNA-Array oder als Protein-Array.
